# EUROPEAN PATENT APPLICATION

(11) **EP 4 252 541 A1**
(43) Date of publication of application: **04.10.2023**
(21) Application number: 21910774.5
(22) Date of filing: 21.12.2021
(51) Int. Cl.: A23K 10/18, A23K 20/163, A23K 50/75

(54) **METHOD FOR IMPROVING PRODUCTIVITY IN LIVESTOCK AND/OR POULTRY AND METHOD FOR IMPROVING INTESTINAL FLORA IN LIVESTOCK AND/OR POULTRY**

(30) Priority: 25.12.2020 JP 2020217886
(71) Applicant: Hayashibara Co., Ltd., Okayama-shi, Okayama 702-8006 (JP)
(72) Inventor: MUKAI Kazuhisa, Okayama-shi Okayama 702-8006 (JP); YAMAMOTO Koryu, Okayama-shi Okayama 702-8006 (JP)
(74) Representative: Page White Farrer
(86) International application number: PCT/JP2021/047296
(87) International publication number: WO 2022/138631

(57) **Abstract**

The present invention aims to provide means capable of improving the intestinal flora and the productivity of livestock and/or poultry, especially means for improving the weight gain, the feed conversion ratio, and the uniformity of livestock and/or poultry. The object is achieved by providing a method of improving the productivity of livestock and/or poultry, and a method of improving the intestinal flora of livestock and/or poultry, by feeding the livestock and/or poultry with a feed containing trehalose and probiotics, and providing a feed for livestock and/or poultry containing trehalose and probiotics.

## Description

### TECHNICAL FIELD

The present invention relates to a method of improving the productivity of livestock and/or poultry, and a method of improving the intestinal flora of livestock and/or poultry. More specifically, the present invention relates to a method of improving the productivity of livestock and/or poultry, and a method of improving the intestinal flora of livestock and/or poultry, by feeding the livestock and/or poultry with a feed containing trehalose and probiotics.

### BACKGROUND ART

Recently in Japan, westernization of the dietary habit has led to an increased demand for animal proteins such as meats, eggs, and milks. Therefore, the livestock industry addresses improvement of the production and the production efficiency of beef, pork, chicken, and the like. In view of this, efficient rearing methods for livestock and poultry, feeds for improving the productivity, and the like are demanded.

For example, in Japan, where the land area is limited, an increase in the productivity was attempted by increasing the number of livestock (poultry) per unit area. However, the productivity has been adversely affected by increased stress on the livestock and poultry caused by their rearing under overcrowded conditions. In addition, the heat in the summer period, the cold in the winter period, poor sanitary conditions of livestock barns, and the like are causing stress, leading to the problem of decreased productivity due to occurrence of diseases, decreased growth rates, and the like.

Moreover, not only in Japan, but also in other countries, it has been pointed out that there are a number of problems in the productivity caused by rearing under overcrowded conditions, such as decreased activity and decreased weight gain of livestock or poultry. Regarding poultry, rearing under overcrowded conditions has been especially problematic in chickens for meat production (broiler chickens). In Europe, "EU directive: minimum rules for the protection of chickens kept for meat production" prescribes that the stocking density of chickens for meat production should not exceed 33 to 42 kg/m² (which corresponds to about 11 to 14 individuals/m²). Although there is no legal regulation in Japan, the practical upper limit of the stocking density before shipping is about 47 kg/m² (which corresponds to about 15 individuals/m²) based on self-regulation by those skilled in the art.

In a high-stress environment due to rearing under overcrowded conditions or the like, the intestinal flora of livestock or poultry tends to be unbalanced because of, for example, a decreased ratio of useful microbial cells and an increased ratio of harmful microbial cells in the intestine. Such unbalanced conditions are known to deteriorate development and homeostasis of the gastrointestinal tract, to cause a decrease in the absorption efficiency of nutrient components, and also known to increase susceptibility to infectious agents such as intestinal putrefactive bacteria, diarrheal bacteria, and diarrheal viruses, resulting in occurrence of enteritis, diarrhea, or dysentery. Once an infection occurs in a livestock barn under overcrowded conditions, the infection spreads in the whole livestock barn, resulting in a further decrease in the productivity, which is problematic.

Examples of harmful microbes that are known to cause such an infection include pathogenic *Escherichia coli,* bacteria belonging to the genus *Salmonella,* bacteria belonging to the genus *Clostridium,* and bacteria belonging to the genus *Campylobacter.* It is known that, when abnormal growth of harmful microbes occurs, toxins such as enterotoxin and cytotoxin are produced to cause disorder of the intestinal mucosa, resulting in occurrence of an infection. In particular, *Clostridium perfringens,* which is a bacterium belonging to the genus *Clostridium* and also called Welch bacillus, is known to be a causative bacterium of necrotic enteritis in livestock and poultry. Welch bacillus is widely distributed in nature, for example, in the intestinal tracts of humans and animals, soils, and water. Welch bacillus is also detected from feces of livestock and poultry, and from fish and the like. Regarding foods, Welch bacillus often adheres to meat such as beef, pork, or chicken, and is known to be a cause of food poisoning. In the livestock industry, Welch bacillus is regarded as a harmful microbe that adversely affects health of livestock and poultry, and hence severely affects their producers from the economic aspect.

Feeds and additive compositions for feeds, for the purpose of preventing or ameliorating diseases of livestock and poultry have been disclosed. For example, Patent Document 1 discloses a growth-promoting agent for birds and mammals, and a feed additive for improving fecal properties of animals, which agent and feed additive contain trehalose. Patent Document 2 discloses a heat stress-reducing agent for livestock, which agent contains trehalose. However, these disclosures are merely based on studies carried out by adding trehalose in a feed, and investigating the weight gain ratio of broiler chickens fed with the feed, or the average feed intake of cows fed with the feed. Therefore, regarding the effect of feeding with trehalose on the productivity, no detailed study was carried out except for the weight gain and the feed intake.

For feeds and feed additives for livestock and poultry, probiotics are used for the purpose of improving the intestinal environment of the livestock and poultry, and promoting their growth. Probiotics are defined as live microbial additives that improve the balance of the intestinal bacteria in a host animal to produce an effective action on the host. The live microbial additives are now generally called probiotics irrespective of their forms (Non-Patent Document 1).

As probiotics for humans and animals, microorganisms belonging to the genus *Lactobacillus,* the genus *Streptococcus,* the genus *Bifidobacterium,* the genus *Bacillus,* the genus *Clostridium,* or the like are used. These are used individually or as mixed formulations (Non-Patent Document 2). Patent Document 3 discloses a method of feeding poultry with the combination of *Bacillus coagulans* and *Bacillus subtilis* during the late stage of the growth. However, there is no concrete description on the effect of the feeding with the probiotics on the intestinal flora.

### PRIOR ART DOCUMENTS

### [Patent Documents]

[Patent Document 1] JP 2008-19236 A
[Patent Document 2] JP 2015-140347 A
[Patent Document 3] JP 6643319 B

### [Non-patent Documents]

[Non-Patent Document 1] Sustainable livestock production and human welfare vol. 66 (12), pp.1189-1196 (2012)
[Non-Patent Document 2] Bifidus vol. 5 (1), pp. 1-18 (1991)

### SUMMARY OF THE INVENTION

### PROBLEMS TO BE SOLVED BY THE INVENTION

An object of the present invention is to provide means capable of improving the intestinal flora and the productivity of livestock and/or poultry, especially means for improving the weight gain, the feed conversion ratio, and the uniformity of livestock and/or poultry.

### MEANS FOR SOLVING THE PROBLEMS

In order to solve the above problem, the present inventors intensively studied to unexpectedly discover that, by feeding livestock and/or poultry with a feed containing trehalose and probiotics, the intestinal flora of the livestock and/or poultry can be improved, and the weight gain, the feed conversion ratio, and the uniformity of the livestock and/or poultry can be effectively improved, thereby completing the present invention.

Thus, the present invention solves the above problem by providing a method of improving the productivity of livestock and/or poultry by feeding the livestock and/or poultry with a feed containing trehalose and probiotics, and a method of improving the intestinal flora of livestock and/or poultry by feeding the livestock and/or poultry with the feed.

### EFFECT OF THE INVENTION

The method of improving the productivity of livestock and/or poultry, and the method of improving the intestinal flora of livestock and/or poultry, of the present invention are capable of reducing the number of harmful microbial cells in the intestine of the livestock and/or poultry, to improve the intestinal flora, and the methods also produce an effect that improves the weight gain, the feed conversion ratio, and the uniformity of the livestock and/or poultry. Therefore, improvement of the productivity of the livestock and/or poultry is possible by the methods.

### BRIEF DESCRIPTION OF THE DRAWINGS

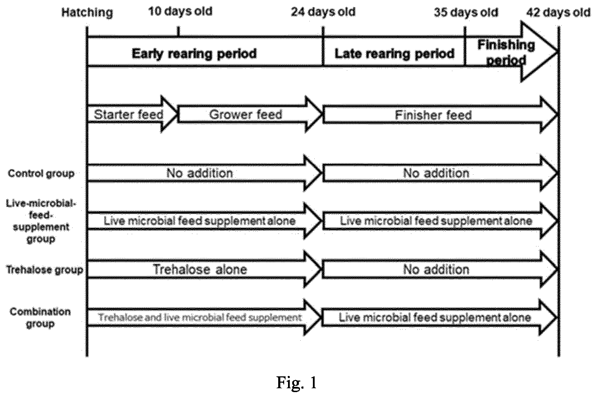

Fig. 1 is a diagram illustrating an example of the stages during the rearing period from hatching to shipping (from 0 day old to 42 days old) of chickens for meat production (broiler chickens), and examples of the feeds given during the period.

### MODE FOR CARRYING OUT THE INVENTION

The present invention relates to a feed containing trehalose and probiotics, and also relates to a method of improving the productivity of livestock and/or poultry, and a method of improving the intestinal flora of livestock and/or poultry, the method including feeding the livestock and/or poultry with a feed containing trehalose and probiotics.

The term "improvement of the productivity of livestock and/or poultry" in the present invention means improvement of the efficiency of obtaining adults that satisfy a certain criterion for the shipment in rearing of the livestock and/or poultry. The improvement of the productivity of livestock and/or poultry may be, for example, weight gain, reduction of the feed conversion ratio, and/or improvement of the uniformity of the livestock and/or poultry.

The weight gain means an increase in the body weight of an animal during a certain rearing period. The larger the value of the weight gain during a certain rearing period, the faster the growth of the animal. Thus, in a certain embodiment, the improvement of the productivity may be a larger value of weight gain during a certain rearing period compared to a case where the animal is fed with a feed containing neither trehalose nor the probiotics, or with a feed not containing one of these.

The feed conversion ratio means the ratio between the feed intake (consumption) by an animal and the weight gain of the animal, in other words, the feed intake (kg) / the weight gain (kg), and hence means the amount of feed required for increasing the body weight of the animal by 1 kg. Thus, the lower the value of the feed conversion ratio, the higher the rate of utilization of the feed by the animal, that is, the better the economic efficiency. Therefore, in a certain embodiment, the improvement of the productivity may be a smaller value of the feed conversion ratio compared to a case where the animal is fed with a feed containing neither trehalose nor the probiotics, or with a feed not containing one of these.

The uniformity means a state where animal individuals show only small variations in the body weight and/or the like, and hence have similar and uniform characteristics. It has been said that, by increasing the uniformity, the meat productivity and the feed utility rate can be improved. In general, the coefficient of variation is used as an index representing the uniformity. The coefficient of variation herein is a value calculated by (standard deviation / average) × 100. The lower the value of the coefficient of variation, the smaller the variation among the animal individuals, that is, the better the uniformity. In the present application, the uniformity regarding the body weight was investigated. Thus, in a certain embodiment, the improvement of the productivity may be improvement of the uniformity of the body weight compared to a case where the animal is fed with a feed containing neither trehalose nor the probiotics, or with a feed not containing one of these.

The term "improvement of the intestinal flora" of livestock and/or poultry in the present invention means maintenance of appropriate conditions of the microbial flora grown in the intestinal tract of the livestock and/or poultry, in other words, an increase in the number of of useful microbial cells and/or a decrease in the number of harmful microbial cells in the intestinal flora. Examples of known useful microbes in the intestinal flora include bacteria belonging to the genus *Lactobacillus,* bacteria belonging to the genus *Bifidobacterium,* bacteria belonging to the genus *Enterococcus,* bacteria belonging to the genus *Streptococcus,* bacteria belonging to the genus *Pediococcus,* bacteria belonging to the genus *Leuconostoc,* bacteria belonging to the genus *Lactococcus,* and bacteria belonging to the genus *Bacillus.* Examples of known harmful microbes in the intestinal flora include, as described above, *Escherichia coli,* bacteria belonging to the genus *Salmonella,* bacteria belonging to the genus *Clostridium,* and bacteria belonging to the genus *Campylobacter.* Thus, the term "improvement of the intestinal flora" of livestock and/or poultry in the present description may include an increase in the number of cells of one or more selected from bacteria belonging to the genus *Lactobacillus,* bacteria belonging to the genus *Bifidobacterium,* bacteria belonging to the genus *Enterococcus,* bacteria belonging to the genus *Streptococcus,* bacteria belonging to the genus *Pediococcus,* bacteria belonging to the genus *Leuconostoc,* bacteria belonging to the genus *Lactococcus,* and bacteria belonging to the genus *Bacillus,* which are useful microbes; and/or a decrease in the number of cells of one or more selected from *Escherichia coli,* bacteria belonging to the genus *Salmonella,* bacteria belonging to the genus *Clostridium,* and bacteria belonging to the genus *Campylobacter,* which are harmful microbes. It is known that, when a stress from the rearing conditions or rearing environment causes abnormality of the intestinal flora of livestock and/or poultry, the ratio of harmful microbial cells such as *Escherichia coli, Salmonella,* and *Clostridium* increases, and the ratio of the number of useful microbial cells decreases, leading to an unbalanced intestinal flora, which adversely affects the livestock and/or poultry in various ways. In particular, *Clostridium perfringens* (hereinafter referred to as "Welch bacillus"), which is known to be a harmful microbe, causes diseases such as necrotic enteritis, gangrenous dermatitis, and cholangiohepatitis in livestock and/or poultry, leading to adverse effects such as a decrease in the productivity and an increase in the mortality. Therefore, reduction of the number of harmful microbial cells such as Welch bacillus cells in the intestine is very important also from the viewpoint of improving the productivity of livestock and/or poultry. Thus, in one preferred mode, the term "improvement of the intestinal flora" of livestock and/or poultry may be reduction of the number of Welch bacillus cells in the intestinal tract of the livestock and/or poultry.

The "livestock" in the present invention is not limited as long as it is an animal genetically improved from a wild animal, in order to utilize it in the human life. Examples of the livestock may include cows, pigs, sheep, goats, horses, dogs, and cats. Examples of the cows include Holstein cattle and Jersey cattle that are dairy cattle; and Aberdeen Angus cattle, Japanese Black cattle, Japanese Brown cattle, crossbred cattle, and Holstein cattle that are cattle for meat production. Examples of the pigs include Landrace pigs, Yorkshire pigs, Hampshire pigs, and Berkshire pigs. Examples of the sheep include Southdown sheep, Suffolk sheep, and Cheviot sheep as sheep for meat production. "Poultry" is not limited as long as it is a bird reared for meat production or egg collection. Examples of the poultry include chickens, quails, turkeys, ducks, geese, mallards, aigamo (hybrids between wild and domestic ducks), and pheasants. Examples of the chickens include broiler chickens, which are chickens for meat production, and layer chickens, which are chickens for egg collection. Examples of breeds of the broiler chickens include Chunky, Cobb, and Arbor Acre.

In a method of improving the productivity and/or a method of improving the intestinal flora according to one mode of the present invention, the period for feeding the livestock and/or poultry with the feed containing trehalose and the probiotics is not limited. The feed may be fed in any period during the rearing period of the livestock and/or poultry. The rearing period of the livestock and/or poultry is the period from birth or hatching to the day of shipping. The length of the period varies among animal species. For example, among livestock species, it is about 30 months in cases of cows for meat production, and is about 190 days in cases of pigs. Regarding poultry, the length of the period is about 25 days to about 170 days. For example, it is about 35 days to about 60 days in cases of broiler chickens. In the later-described Experimental Examples, the rearing period in the rearing test of broiler chickens was set to the period of 42 days from hatching to 42 days old.

In livestock, the type of the feed to be given varies depending on the animal species and the rearing stage. Therefore, the feed containing trehalose and the probiotics may be provided in various forms depending on the type and the rearing stage of the animal to be fed. For example, livestock are mainly fed with two kinds of feeds: a fiber-rich coarse feed such as grass, hay, or silage, and a concentrated feed containing a large amount of protein and carbohydrate such as cereals, soybean, or rice bran. However, cows in the sucking period immediately after birth are fed with a milk substitute, which contains non-fat dry milk as the main raw material, and which is given in the form of a liquid prepared by dissolving the non-fat dry milk, or fed with an artificial milk, which is a concentrated feed to be given in the form of a solid. Thus, in a certain embodiment, the feed according to the present invention may be a coarse feed, a concentrated feed, a milk substitute, or an artificial milk. The rearing period can be roughly divided into the first half and the second half. The first half may be referred to as "early rearing period", and the latter half may be referred to as "late rearing period". Usually, the period called "sucking period", which corresponds to the period from birth to weaning, is called "early rearing period", and the period called "growing period" and/or "fattening period", which corresponds to the period from after weaning to the day of shipping, is called "late rearing period", although the periods may vary depending on the type of livestock. As described above, the period of feeding the livestock with a feed containing trehalose and the probiotics according to one mode of the present invention is not limited. Since a sufficient effect can be obtained by feeding the livestock with the feed in the form of a milk substitute and/or an artificial milk to be given during the sucking period (early rearing period), the feed is preferably given during the sucking period (early rearing period). In other words, in a certain embodiment, the feed of the present invention can be especially preferably used as a milk substitute and/or an artificial milk. However, the feeding period and the form of the feed of the present invention are not limited to these. For example, needless to say, the feed may be given at a rearing stage other than the sucking period, and of course, the feed may be used as a coarse feed or a concentrated feed.

In poultry, the nutrient composition of the feed to be given may vary depending on the rearing stage. Therefore, the feed containing trehalose and the probiotics may be provided in various forms depending on the rearing stage of the poultry to be fed. Examples of the feed to be given to the poultry include feeds for chicks called "starter feed", which are rich in nutrients such as amino acids and proteins; feeds for the growth called "grower feed", which contain a larger amount of lipids than the starter feed; and feeds for finishing called "finisher feed". The rearing period can be roughly divided into the first half and the second half. The first half may be referred to as "early rearing period", and the latter half may be referred to as "late rearing period". The early rearing period may vary depending on the type, the rearing conditions, the degree of growth, and the like of the poultry. In cases where the rearing period is 35 days, the early rearing period is usually from 0 day old to 15 to 18 days old. In cases where the rearing period is 42 days, the early rearing period is usually from 0 day old to 20 to 24 days old. In cases where the rearing period is 60 days, the early rearing period is usually from 0 day old to 26 to 34 days old. The remaining rearing period is regarded as the late rearing period. In the early rearing period, a starter feed or grower feed is given to chicks after hatching. In the late rearing period, a finisher feed is given. The seven days before shipping is called "finishing period". During this period, a feed for finishing, which is different from the finisher feed, may be given. The period during which the poultry is fed with a feed according to one mode of the present invention is not limited. Since a sufficient effect can be obtained by feeding the poultry with a feed in the form of a starter feed and/or a grower feed to be given during the early rearing period, the feed according to one mode of the present invention is especially useful as a starter feed and/or a grower feed. The feed of the present invention may be used, of course, as a finisher feed.

In a method of improving the productivity of livestock and/or poultry, and a method of improving the intestinal flora of livestock and/or poultry according to one mode of the present invention, the trehalose used as an effective component of the feed means the non-reducing disaccharide having the structure in which two molecules of D-glucose are bound together through an α,α-1,1 bond (α,α-trehalose).

The trehalose used in the present invention is not limited in terms of its origin or production method. In one preferred mode, a hydrated crystal or an anhydrous crystal of trehalose produced by the method disclosed in JP 7-170977 A, JP 7-213283 A, or the like may be used, when appropriate. More specifically, a commercially available product such as a trehalose dihydrate crystal-containing powder industrially produced from starch by the enzyme method ("TREHA (registered trademark)" or "TREHA (registered trademark) A Feed", commercially available from Hayashibara Co., Ltd.) may be suitably used. The purity of the trehalose used in the present invention is not limited as long as the effect of the present invention can be produced. The trehalose may be a pure product, or may be a mixture with another carbohydrate, that is, an unseparated composition. The form of the trehalose is also not limited, and the trehalose may be in the form of a liquid, syrup, powder, solid, or the like.

The probiotics used in the present invention are described below. The "probiotics" in the present invention means a useful intestinal microorganisms used by allowing an animal to ingest the probiotics by including it in an animal feed, and allowing the microorganism to grow in the intestine of the animal, to maintain favorable conditions of the intestinal flora, or a feed additive containing the useful intestinal microorganism. Examples of the useful intestinal microorganism include microorganisms belonging to the genus *Lactobacillus,* such as *Lactobacillus acidophilus, Lactobacillus salivarius,* and *Lactobacillus casei;* microorganisms belonging to the genus *Bifidobacterium* generally called bifidobacteria, such as *Bifidobacterium thermophilum, Bifidobacterium pseudolongum,* and *Bifidobacterium longum;* microorganisms belonging to the genus *Enterococcus,* such as *Enterococcus faecalis* and *Enterococcus faecium*; microorganisms belonging to the genus *Streptococcus,* such as *Streptococcus thermophilus* and *Streptococcus mutans;* microorganisms belonging to the genus *Pediococcus,* such as *Pediococcus pentosaceus;* microorganisms belonging to the genus *Leuconostoc,* such as *Leuconostoc citreum* and *Leuconostoc mesenteroides;* live microbes generally called lactic acid bacteria, such as those belonging to the genus *Lactococcus,* including *Lactococcus lactis;* microorganisms belonging to the genus *Clostridium,* such as *Clostridium butyricum;* and microorganisms belonging to the genus *Bacillus,* such as *Bacillus subtilis, Bacillus cereus, Bacillus badius,* and *Bacillus coagulans.* These microorganisms, and/or feed additives containing these microorganisms, can be suitably used as probiotics for animal feeds.

Among the above useful intestinal microorganisms, microorganisms belonging to the genus *Lactobacillus* or the genus *Bacillus* are preferred from the viewpoint of the action and effect that improve the intestinal flora of livestock and/or poultry. *Bacillus subtilis* is especially preferred. As the *Bacillus subtilis,* the *Bacillus subtilis* C-3102 strain or a strain derived therefrom, or a mutant strain thereof, is preferred. The form of the probiotics is not limited, and the probiotics may be in the form of any of a liquid, powder, and solid. A commercially available probiotics may also be used. Examples of the commercially available product include "CALSPORIN (registered trademark)", which is probiotics in a powder form commercially available from Asahi Biocycle Co., Ltd., and which contains the *Bacillus subtilis* C-3102 strain (FERM BP-1096) as a major component (the content of the *Bacillus subtilis* C-3102 strain: 1×10¹⁰ cells/g- probiotics); and a variety of related products thereof with different live microbial contents (trade name: "CALSPORIN (registered trademark) Dilutions"). The live microbial content is arbitrarily selected depending on the desired effect.

The amount of trehalose and the number of live microbial cells in a feed according to one mode of the present invention are not limited, and appropriately adjusted depending on the type of the livestock and/or poultry to be fed, the types and amounts of other components included in the feed, the condition of the skin of the livestock and/or poultry to be fed, the condition of the hair, the maturation condition of the individual, the sex, the body weight, and/or the like. The amount of trehalose may be adjusted such that the feed contains trehalose in an amount within the range of usually 0.05% by mass to 2% by mass, more preferably 0.1% by mass to 1% by mass, still more preferably 0.2% by mass to 0.8% by mass. The number of live microbial cells in the feed is preferably adjusted to a ratio of usually about 3.0×10⁵ cells to 1.0×10⁹ cells, more preferably about 5.0×10⁵ cells to 1.0×10⁷ cells per 1 g (gram) of the feed. In cases where the content of trehalose in the feed is less than 0.05% by mass, or where the number of live microbial cells contained in the feed is less than 3.0×10⁵ per 1 g of the feed, the effect to improve the productivity of livestock and/or poultry, and/or the effect to improve the intestinal flora of livestock and/or poultry, may be insufficient. On the other hand, even in cases where the content of trehalose in the feed is over 2% by mass, or where the number of live microbial cells per 1 g of the feed is over 1.0×10⁹, the effects are not largely different from those in the cases where trehalose is added at 2% by mass, or where 1.0×10⁹ live microbial cells are added per 1 g of the feed. Therefore, such cases are not preferred from an economic point of view.

A feed according to one mode of the present invention may contain, in addition to trehalose and the probiotics, a feed raw material or feed additive commonly used for livestock and/or poultry. Examples of the feed raw material or feed additive that may be included in the feed according to one mode of the present invention include, but are not limited to, cereals such as corn, rice, brown rice, foxtail millet, proso millet, rye, cassava, barley, wheat, oat, milo, and soybean; vegetable oil cakes such as soybean oil cake, rapeseed oil cake, linseed oil cake, palm oil cake, sesame oil cake, sunflower oil cake, coconut oil cake, peanut meal, cottonseed meal, corn gluten meal, corn germ meal, and alcohol lee; animal feeds such as fish meal, meat and bone meal, casein, dried whey, non-fat dry milk, and fish soluble-adsorbed feeds; chaff and bran such as rice bran, defatted rice bran, wheat bran, soybean hull, starch cake, barley bran, corn gluten feed, and molasses.

Further, for a feed according to one mode of the present invention, other components such as antioxidants, fungicides, binders, amino acids, vitamins, minerals, pigments, synthetic antimicrobial agents, antibiotics, flavoring agents, enzymes, organic acids, emulsifiers, excipients, sugars, adjusting agents, corrigents, and stabilizers may be used in combination depending on the type and the form of the feed, to an extent that does not prevent the effects to improve the productivity and the intestinal flora of livestock and/or poultry. Examples of those components that may be included in a feed according to one mode of the present invention include the following. Examples of the antioxidants include ethoxyquin, dibutylhydroxytoluene, and butylhydroxyanisole. Examples of the fungicides include propionic acid, calcium propionate, and sodium propionate. Examples of the binders include pullulan, sodium alginate, sodium caseinate, sodium carboxymethyl cellulose, propylene glycol, and sodium polyacrylate. Examples of the amino acids include aminoacetic acid, DL-alanine, L-arginine, L-lysine hydrochloride, L-carnitine, guanidine acetic acid, sodium L-glutamate, taurine, 2-deamino-2-hydroxymethionine, DL-tryptophan, L-tryptophan, L-threonine, L-valine, DL-methionine, L-methionine, and L-lysine sulfate. Examples of the vitamins, include L-ascorbic acid, calcium L-ascorbate, sodium L-ascorbate, sodium calcium L-ascorbic acid-2-phosphate ester, magnesium L-ascorbic acid-2-phosphate ester, acetomenaphthone, inositol, dibenzoylthiamine hydrochloride, ergocalciferol, choline chloride, thiamine hydrochloride, pyridoxine hydrochloride, β-carotene, cholecalciferol, dl-α-tocopherol acetate, cyanocobalamin, thiamine nitrate, nicotinic acid, nicotinic acid amide, para-aminobenzoic acid, calcium D-pantothenate, calcium DL-pantothenate, d-biotin, vitamin A powder, vitamin A oil, vitamin D powder, vitamin D3 oil, vitamin E powder, 25-hydroxycholecalciferol, menadione dimethylpyrimidinol bisulfite, menadione sodium bisulfite, folic acid, riboflavin, and riboflavin butyrate. Examples of the minerals include potassium chloride, iron citrate, calcium gluconate, sodium iron succinate citrate, magnesium oxide, aluminum hydroxide, zinc carbonate, cobalt carbonate, sodium hydrogen carbonate, magnesium carbonate, manganese carbonate, 2-deamino-2-hydroxymethionine zinc, 2-deamino-2-hydroxymethionine copper, 2-deamino-2-hydroxymethionine manganese, DL-threonine iron, calcium lactate, ferrous fumarate, peptide zinc, peptide iron, peptide copper, peptide manganese, potassium iodide, potassium iodate, calcium iodate, zinc sulfate (dry), zinc sulfate (crystal), zinc sulfate methionine, sodium sulfate (dry), magnesium sulfate (dry), magnesium sulfate (crystal), cobalt sulfate (dry), cobalt sulfate (crystal), iron sulfate (dry), copper sulfate (dry), copper sulfate (crystal), manganese sulfate, potassium monohydrogen phosphate (dry), sodium monohydrogen phosphate (dry), potassium dihydrogen phosphate (dry), sodium dihydrogen phosphate (dry), and sodium dihydrogen phosphate (crystal). Examples of the pigments include astaxanthin, ethyl β-apo-8'-carotenoate, and canthaxanthin. Examples of the synthetic antimicrobial agents include amprolium-ethopabate-sulfaquinoxaline, morantel citrate, nicarbazin, and halofuginone calcium polystyrene sulfonate. Examples of the antibiotics include zinc bacitracin, avilamycin, enramycin, salinomycin sodium, semduramycin sodium, narasin, nosiheptide, bicozamycin, flavophospholipol, monensin sodium, lasalocid sodium, and coccidiostat. Examples of the flavoring agents include esters, ethers, ketones, fatty acids, aliphatic higher alcohols, aliphatic higher aldehydes, aliphatic higher hydrocarbons, terpene hydrocarbons, phenol ethers, phenols, aromatic alcohols, aromatic aldehydes, and lactones. Examples of the enzymes include amylase, alkaline protease, xylanase, xylanase-pectinase enzyme complex, β-glucanase, acid protease, cellulase, cellulase-protease-pectinase enzyme complex, neutral protease, phytase, lactase, and lipase. Examples of the organic acids include calcium formate, sodium gluconate, potassium diformate, fumaric acid, butyric acid, lactic acid, and acetic acid, and salts thereof. Examples of the emulsifiers include glycerin fatty acid esters and sorbitan fatty acid esters. Examples of the excipients include corn starch and dextrin. Examples of the sugars include sucrose, glucose, maltose, fructose, lactose, maltotriose, raffinose, isomaltulose, maltitol, reduced palatinose, sorbitol, xylitol, erythritol, mannitol, isomerized sugars, and powdered starch syrups. Examples of the adjusting agents include formic acid. Examples of the corrigents include saccharin sodium. Examples of the stabilizers include silicic anhydride.

The form of the feed according to one mode of the present invention is not limited. For example, the feed may be in the form of a mash feed, granular feed, fine powder feed, flake feed, pellet feed, crumbled feed, or the like. The mash feed is a feed in a powder form prepared by pulverizing a feed raw material into a powder, or by mixing or blending of a powdery feed raw material. The pellet feed is a solid feed prepared by humidifying a feed under heat while adding water thereto using water vapor or the like, and then pressure-molding the humidified feed into a columnar shape using a granulator (molding machine). The crumbled feed is a feed prepared by cracking a pellet-form feed such that the feed can be easily taken by small-sized poultry.

The present invention is described below in more detail based on experiments.

### <Experiment 1: Effect of Feeding with Feed Containing Trehalose and Probiotics on Productivity and Intestinal Flora of Broiler Chickens>

Broiler chickens were fed with a feed containing trehalose and probiotics at each rearing stage, to test the effect of the feeding with the feed on the productivities including the weight gain, the feed conversion ratio, and the uniformity of the broiler chickens, and on the intestinal flora of these chickens.

### <Experiment 1-1: Preparation of Feeds>

Three kinds of feeds, more specifically, a starter feed and a grower feed for the early rearing period, and a finisher feed for the late rearing period and the finishing period, were prepared according to the blend formulation disclosed in Ross Broiler Nutrition Specifications 2019 (URL: http://tmea.aviagen.com/assets/Tech_Center/Ross_Broiler/RossBroilerNutritionSpec s2019-EN.PDF), which is a manual provided by the broiler chicken breeding company Aviagen (located in UK), such that the feeds have the compositions shown in Table 1. The prepared samples were provided as a starter feed containing no additive, a grower feed containing no additive, and a finisher feed containing no additive. To the starter feed containing no additive, and the grower feed containing no additive, trehalose (trade name "TREHA", commercially available from Hayashibara Co., Ltd.) was added at 0.5% by mass, and/or probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) was added at 0.01% by mass (corresponding to 1.0×10⁶ cells/g-feed), to prepare a starter feed containing trehalose, a starter feed containing the probiotics, a grower feed containing trehalose, a grower feed containing the probiotics, and a starter feed and a grower feed each containing both trehalose and the probiotics. Further, the probiotics were added at 0.01% by mass (corresponding to 1.0×10⁶ cells/g-feed), without addition of trehalose, to the finisher feed containing no additive, to prepare a finisher feed containing the probiotics, similarly to the cases of the starter feed containing the probiotics and the grower feed containing the probiotics.

**[Table 1]**

| Component | Starter feed | Grower feed | Finisher feed |
|---|---|---|---|
| Corn (7.6% crude protein) | 54.62 | 57.17 | 59.62 |
| Dehulled soybean oil cake (49.4% crude protein) | 37.07 | 33.93 | 30.55 |
| Soybean oil | 3.33 | 4.450 | 5.58 |
| Mono-dicalcium phosphate (18.5% calcium, 22% phosphorus) | 1.940 | 1.726 | 1.601 |
| Limestone (38.8% calcium) | 1.198 | 1.095 | 1.029 |
| Pellet binder (Pelex Dry) | 0.300 | 0.300 | 0.300 |
| Common salt | 0.217 | 0.240 | 0.240 |
| Vitamin premix for broiler chickens | 0.200 | 0.200 | 0.200 |
| DL-Methionine | 0.328 | 0.277 | 0.269 |
| L-Lysine hydrochloride | 0.226 | 0.162 | 0.157 |
| L-Threonine | 0.127 | 0.081 | 0.069 |
| Sodium bicarbonate (27% sodium) | 0.231 | 0.201 | 0.204 |
| Choline chloride (50% choline) | 0.111 | 0.103 | 0.111 |
| Salinomycin | 0.050 | 0.050 | 0.050 |
| L-Isoleucine | 0.044 | 0.014 | 0.019 |
| Total | 100 | 100 | 100 |

| | | | |
|---|---|---|---|
| Unit: % by mass | | | |

### <Experiment 2: Rearing Test>

A rearing test was carried out using chicks of male Chunky broiler chickens at a stocking density of 11 or 14 individuals per test area (1 m²). The chicks were divided into the four groups shown in Table 2 and Fig. 1 depending on whether or not trehalose and/or the probiotics was/were added to their feeds. For each group, eight sets of test areas were provided, and the two kinds of stocking densities were tested in each set of the test areas. Thus, a total of 64 test areas were set. In Table 2, "+" indicates that trehalose or the probiotics was added to the feed, and "-" indicates that it was not added, in each group. As shown in Fig. 1, the chickens were reared for a total of 42 days, during which a starter feed in a crumbled form was given from hatching to 10 days old; a grower feed in a pellet form was given from 10 days old to 24 days old; and a finisher feed in a pellet form was give from 24 days old to 42 days old. The feeding was carried out at the two kinds of stocking densities. One tubular feeder was used for each test area, and the chickens were provided with ad libitum access to the feed. The chickens were also provided with ad libitum access to water using three nipple drinkers per test area. Rice husk was used as bedding. Regarding the other rearing conditions, those in accordance with Ross Broiler Magagement Handbook 2018 (published from Aviagen, URL: http://en.aviagen.com/assets/Tech_Center/Ross_Broiler/Ross-BroilerHandbook2018-EN.pdf) were employed. The rearing was carried out in a closed poultry house equipped with tunnel ventilation and an evaporative cooling system.

**[Table 2]**

| | Starter feed and grower feed | | Finisher feed | |
|---|---|---|---|---|
| | Trehalose | Probiotics | Trehalose | Probiotics |
| Control group | - | - | - | - |
| Probiotics group | - | + | - | + |
| Trehalose group | + | - | - | - |
| Combination group | + | + | - | + |

| | | | | |
|---|---|---|---|---|
| *Trehalose was added only to the starter feed and the grower feed. | | | | |

### <Experiment 3: Intestinal Flora>

In order to evaluate the intestinal flora of the broiler chickens reared in each test area, one broiler chicken having a body weight close to the average in each group at 24 days old was selected from each test area of each group. The ileum content was then collected from the broiler chicken. The collected ileum content was subjected to measurement of the number of cells of bacteria belonging to the genus *Lactobacillus,* which is known to be a useful microbe, and the number of cells of Welch bacillus (*Clostridium perfringens*), which is known to be a harmful microbe. Table 3 shows the numbers of the cells per unit weight of the ileum content. Comparison focusing on the stocking density was carried out between the environment at 11 individuals/m², which corresponds to a normal stocking density for broiler chickens, and the rearing environment at 14 individuals/m², which corresponds to a relatively high density. As a result, although a more evident decrease in the number of cells of Welch bacillus was found under the high-density rearing condition of 14 individuals/m², no remarkable difference was found between these different stocking densities. Therefore, in Table 3, the average of the measurement results from the two kinds of stocking densities is shown. This also applies to the later-described Experiments 4 and 5. Since no remarkable difference was found between the two kinds of stocking densities also in terms of the weight gain, the feed conversion ratio, and the uniformity, their measurement values are shown as the averages of the measurement results obtained at the two kinds of stocking densities. The number of cells of bacteria belonging to the genus *Lactobacillus,* and the number of cells of Welch bacillus, in each of a control group and the test groups were tested by Duncan's multiple range test. Significance was assumed at a significance level of p<0.05. The symbols " * " in the tables represent the presence of a significant difference compared to the control group.

**[Table 3]**

| | Number of microbial cells (cells/g) | |
|---|---|---|
| | *Lactobacillus spp.* | *Clostridium perfringens* |
| Control group | 4.7 × 10⁷ | 3.8 × 10² |
| Probiotics group | 9.3 × 10⁷ | 1.1 × 10^{2∗} |
| Trehalose group | 10.2 × 10⁷ | 0.37 × 10^{2∗} |
| Combination group | 15.1 × 10⁷ | 0.22 × 10^{2∗} |

As can be seen in Table 3, in the ileum content collected from the broiler chickens at 24 days old, the number of cells of bacteria belonging to the genus *Lactobacillus* per unit weight (cell/g) was 4.7×10⁷ cells/g in the control group. In contrast, the number of these cells was 9.3×10⁷ cells/g in the probiotics group, 10.2×10⁷ cells/g in the trehalose group, and 15.1×10⁷ cells/g in the combination group. Thus, all of these groups showed increases compared to the control group, and the number of cells of bacteria belonging to the genus *Lactobacillus,* which are useful microbes, was the largest in the combination group.

As can be further seen in Table 3, the number of cells of Welch bacillus (cell/g) was 3.8×10² cells/g in the control group. In contrast, the number of these cells was 1.1×10² cells/g in the probiotics group, and 0.37×10² cells/g in the trehalose group, showing significant decreases compared to the control group. Further, the number of these cells in the combination group was 0.22×10² cells/g, showing a significant decrease compared to the control group. The combination group showed a more remarkable decrease in the number of cells of Welch bacillus even compared to the probiotics group and the trehalose group. These results indicate that combined use of trehalose and the probiotics, as compared to their individual uses, is more effective not only for increasing the number of cells of bacteria belonging to the genus *Lactobacillus,* which are useful microbes, but also for remarkably decreasing the number of cells of Welch bacillus, which is a harmful microbe. Thus, the results suggest that the coexistence of trehalose and the probiotics in the feed is more useful for the improvement of the intestinal flora of poultry, especially broiler chickens.

### <Experiment 4: Weight Gain and Feed Conversion Ratio>

For the broiler chickens in each test area, the body weight was measured at each of 0 day old, 35 day old, and 42 day old, and the average body weight at each age was calculated. The results are shown in Table 4. The weight gain during each period was calculated from the differences between these average body weights. The feed conversion ratio was calculated by dividing the feed intake during the period of from 0 day old to 42 days old, from 0 day old to 35 days old, or from 35 days old to 42 days old, by the weight gain during the corresponding period. The feed intake was determined by measuring the decrease in the amount of the feed during the corresponding period in each test area, and calculating the average. Table 5 to Table 7 show the weight gain, the feed intake, and the feed conversion ratio during the period of from 0 day old to 42 days old, during the period of from 0 day old to 35 days old, and during the period of from 35 days old to 42 days old. The weight gain and the feed conversion ratio in each of the control group and the test groups were tested by Duncan's multiple range test. Significance was assumed at a significance level of p<0.05. The symbols " * " in the tables represent the presence of a significant difference compared to the control group.

**[Table 4]**

| | Average body weight (g) | | |
|---|---|---|---|
| | 0 day old | 35 days old | 42 days old |
| Control group | 41 | 2605 | 3198 |
| Probiotics group | 41 | 2610 | 3213 |
| Trehalose group | 41 | 2621 | 3251 |
| Combination group | 41 | 2650 | 3278 |

**[Table 5]**

| | From 0 day old to 42 days old (entire rearing period) | | |
|---|---|---|---|
| | Weight gain (g) | Feed intake (g) | Feed conversion ratio |
| Control group | 3157 | 4711 | 1.492 |
| Probiotics group | 3172 | 4665 | 1.471* |
| Trehalose group | 3210* | 4756 | 1.482* |
| Combination group | 3237* | 4748 | 1.467* |

As can be seen in Table 5, the weight gain was 3157 g in the control group during the entire rearing period of from 0 day old to 42 days old. The weight gain in the probiotics group was 3172 g. Although this group showed a slight increase compared to the control group, the increase was not a significant change. In contrast, the weight gain was 3210 g in the trehalose group, and 3237 g in the combination group, showing significant increases compared to the control group. Although the feeding with trehalose was limited to the early rearing period of from 0 day old to 24 days old, the trehalose group and the combination group showed a weight gain-promoting effect throughout the entire rearing period of 0 day old to 42 days old. It was thus shown that the effect by the feeding with trehalose during the early rearing period continued until 42 days old in both groups. In particular, the combination group showed an even larger increase in the body weight compared to the trehalose group, indicating that feeding with a feed containing both trehalose and the probiotics during the early rearing period is extremely effective for the weight gain throughout the entire rearing period. Even simply from the viewpoint of the weight gain, this result indicates that feeding with the feed containing both trehalose and the probiotics does not necessarily need to be carried out throughout the entire rearing period, and that the feeding period may be limited to the early rearing period. This is an extremely useful discovery.

As can be further seen in Table 5, the feed conversion ratio, which is calculated from the weight gain and the feed intake, was 1.492 in the control group. In contrast, the feed conversion ratio was 1.471 in the probiotics group, 1.482 in the trehalose group, and 1.467 in the combination group, showing significant decreases compared to the control group. Although the feeding with the combination of trehalose and the probiotics was limited to the early rearing period, the decreases in the feed conversion ratio were found throughout the entire rearing period. It was thus shown that the effect to decrease the feed conversion ratio by the feeding with trehalose and the probiotics during the early rearing period continued until 42 days old. In particular, the feed conversion ratio was lowest in the combination group. Similarly, to the result on the weight gain, this result, from the viewpoint of the feed conversion ratio, suggests that feeding with the feed containing both trehalose and the probiotics does not necessarily need to be carried out throughout the entire rearing period, and that the feeding period may be limited to the early rearing period.

**[Table 6]**

| | From 0 day old to 35 days old (early rearing period + late rearing period) | | |
|---|---|---|---|
| | Weight gain (g) | Feed intake (g) | Feed conversion ratio |
| Control group | 2564 | 3450 | 1.346 |
| Probiotics group | 2569 | 3418 | 1.330* |
| Trehalose group | 2580 | 3458 | 1.340 |
| Combination group | 2609 | 3459 | 1.326* |

As can be seen in Table 6, the weight gain was 2564 g in the control group during the period of from 0 day old to 35 days old. Further, the weight gain was 2569 g in the probiotics group, and 2580 g in the trehalose group. Thus, although both groups showed increases compared to the control group, the amounts of increase were small. In contrast, the weight gain in the combination group was 2609 g, so that a higher level of weight gain was found compared to the other two groups.

As can be further seen in Table 6, the feed conversion ratio was 1.346 in the control group. Further, the feed conversion ratio in the trehalose group was 1.340, showing no significant decrease compared to the control group. In contrast, the feed conversion ratio was 1.330 in the probiotics group, and 1.326 in the combination group, showing significant decreases compared to the control group. The decrease in the feed conversion ratio was largest in the combination group. This result suggests that, although the feeding with the combination of trehalose and the probiotics was limited to the early rearing period (from 0 day old to 24 days old), its effect continued until 35 days old, which is in the late rearing period.

**[Table 7]**

| | From 35 days old to 42 days old (finishing period) | | |
|---|---|---|---|
| | Weight gain (g) | Feed intake (g) | Feed conversion ratio |
| Control group | 593 | 1284 | 2.165 |
| Probiotics group | 603 | 1273 | 2.111* |
| Trehalose group | 630* | 1320 | 2.095* |
| Combination group | 628* | 1308 | 2.083* |

As can be seen in Table 7, the weight gain was 593 g in the control group during the finishing period of from 35 days old to 42 days old. Further, the weight gain in the probiotics group was 603 g, showing a slight increase compared to the control group. In contrast, the weight gain was 630 g in the trehalose group, and 628 g in the combination group, showing significant increases compared to the control group. Although the feeding with the feed containing trehalose or the feed containing both trehalose and the probiotics was limited to the early rearing period of from 0 day old to 24 days old, the trehalose group and the combination group showed a weight gain-promoting effect also during the finishing period of from 35 days old to 42 days. This result suggests the unexpected fact that the effect of the feed given during the early rearing period continues to the finishing period.

As can be further seen in Table 7, the feed conversion ratio was 2.165 in the control group. In contrast, the feed conversion ratio was 2.111 in the probiotics group, 2.095 in the trehalose group, and 2.083 in the combination group. Thus, significant decreases were found in all of these groups compared to the control group. In particular, the feed conversion ratio was lowest in the combination group. This result suggests that, although the feeding with the feed containing both trehalose and the probiotics was limited to the early rearing period, the effect to decrease the feed conversion ratio continued also during the finishing period of from 35 days old to 42 days old.

As described above, the results shown in Table 5 to Table 7 suggest that combined use of trehalose and the probiotics in the early rearing period is useful for increasing the body weight and reducing the feed conversion ratio of broiler chickens throughout the entire rearing period. In particular, combined use of trehalose and the probiotics in the early rearing period is economically advantageous in rearing of broiler chickens since the feed conversion ratio can be reduced even during the finishing period before shipping, when the broiler chickens have high body weight and show maximum feed intake.

### <Experiment 5: Uniformity>

The uniformity of the broiler chickens in each test area was evaluated using as an index the coefficient of variation calculated from the body weights of the individual broiler chickens at 42 days old. The results are shown in Table 8. The uniformity in each of the control group and the test groups was tested by Duncan's multiple range test. Significance was assumed at a significance level of p<0.05. The symbols " * " in the table represent the presence of a significant difference compared to the control group.

**[Table 8]**

| | Coefficient of variation (%) |
|---|---|
| Control group | 8.8 |
| Probiotics group | 7.2 * |
| Trehalose group | 7.9 |
| Combination group | 7.0* |

As can be seen in Table 8, the coefficient of variation as an index of the uniformity was 8.8% in the control group. Further, the coefficient of variation was 7.9% in the trehalose group. Although a slight decrease was found in this group compared to the control group, the decrease was not a significant change. In contrast, the coefficient of variation was 7.2% in the probiotics group, and 7.0% in the combination group, showing significant decreases compared to the control group. Since the combination group showed the lowest coefficient of variation, it was suggested that combined use of trehalose and the probiotics is useful for improvement of the uniformity. Although the feeding with the feed containing both trehalose and the probiotics was limited to the early rearing period, the combination group showed the best result on the uniformity at the end of the rearing period compared to the control group, the probiotics group, and the trehalose group. This fact was totally unexpected, and was a novel discovery.

Thus, from the results of Experiments 1 to 5, it was found that, in rearing of broiler chickens, the intestinal flora of the broiler chickens can be improved, and their productivity can also be improved, by feeding the broiler chickens with a feed using the combination of trehalose and the probiotics during the early rearing period.

The present invention is described below more concretely by way of Examples. However, the present invention is not limited by these Examples.

### EXAMPLE 1

A mixed feed for chicks was prepared by adding 0.5 g of trehalose (trade name "TREHA", commercially available from Hayashibara Co., Ltd.) and 0.03 g of probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) (corresponding to a number of live microbial cells of 3.0×10⁵ cells/g-feed) to 1 kg of a commercially available feed for chicks. Further, a mixed feed for adult chickens was prepared by adding the probiotics to a commercially available feed for adult chickens such that the same amount of the probiotics was included. Since these mixed feeds contain trehalose and the probiotics, feeding of poultry with the feeds during the corresponding rearing stages enables reduction of harmful microbial cells in the intestinal flora of the poultry, and improvement of the productivity of the poultry.

### EXAMPLE 2

A mixed feed for chicks was prepared by adding 10.0 g of trehalose (trade name "TREHA", commercially available from Hayashibara Co., Ltd.) and 10.0 g of probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) (corresponding to a number of live microbial cells of 1.0×10⁹ cells/g-feed) to 1 kg of a commercially available feed for chicks. Further, a mixed feed for adult chickens was prepared by adding the probiotics to a commercially available feed for adult chickens such that the same amount of the probiotics was included. Since these mixed feeds contain trehalose and the probiotics, feeding of poultry with the feeds during the corresponding rearing stages enables reduction of harmful microbial cells in the poultry, and improvement of the productivity of the poultry.

### EXAMPLE 3

A mixed feed was prepared by adding trehalose (trade name "TREHA", commercially available from Hayashibara Co., Ltd.) and probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) to a feed in a powder form that is a mixture of 61.5 parts by mass of corn, 29.0 parts by mass of soybean meal, 2.0 parts by mass of rapeseed meal, 2.0 parts by mass of corn gluten meal, 2.8 parts by mass of animal oil and fat, 0.3 parts by mass of common salt, 1.4 parts by mass of calcium carbonate, 0.5 part by mass of tribasic calcium phosphate, and 0.5 part by mass of a vitamin-mineral premix, such that the trehalose content in the feed solid component was 0.8% by mass, and the number of live microbial cells per 1 g of the feed solid was 1.0×10⁷. Since this product contains trehalose and the probiotics, it enables reduction of harmful microbial cells in the intestinal flora of poultry, and improvement of the productivity of the poultry.

### EXAMPLE 4

A mixed feed was prepared by adding trehalose (trade name "TREHA", commercially available from Hayashibara Co., Ltd.) and probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) to a feed in a powder form that is a mixture of 61.5 parts by mass of corn, 29.0 parts by mass of soybean meal, 2.0 parts by mass of rapeseed meal, 2.0 parts by mass of corn gluten meal, 2.8 parts by mass of animal oil and fat, 0.3 parts by mass of common salt, 1.4 parts by mass of calcium carbonate, 0.5 part by mass of tribasic calcium phosphate, and 0.5 part by mass of a vitamin-mineral premix, such that the trehalose content in the feed solid component was 0.2% by mass, and the number of live microbial cells per 1 g of the feed solid was 5.0×10⁵. Since this product contains trehalose and the probiotics, it enables reduction of harmful microbial cells in poultry, and improvement of the productivity of the poultry.

### EXAMPLE 5

A mixture was prepared by adding 0.5 g of trehalose (trade name "TREHA", commercially available from Hayashibara Co., Ltd.) and 0.03 g of probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) (corresponding to a number of live microbial cells of 3.0×10⁵ cells/g-feed) to 1 kg of a commercially available feed for poultry, and the resulting mixture was fed into a pellet mill that is a heating granulator, to obtain a granulated product. The granulated product was dried to prepare a pellet feed having a cylindrical shape. Since this product is in a pellet form, powder dispersion in air can be prevented; excellent storage stability can be achieved; and the product can be easily handled even in cases where it is in a small amount. Furthermore, since the product contains trehalose and the probiotics, it enables reduction of harmful microbial cells in the intestinal flora of poultry, and improvement of the productivity of the poultry.

### EXAMPLE 6

A mixture was prepared by adding 10.0 g of trehalose (trade name "TREHA", commercially available from Hayashibara Co., Ltd.) and 10.0 g of probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) (corresponding to a number of live microbial cells of 1.0×10⁹ cells/g-feed) to 1 kg of a commercially available feed for poultry, and the resulting mixture was fed into a pellet mill that is a heating granulator, to obtain a granulated product. The granulated product was dried, and crushed by a crumbler, to prepare a crumbled feed. Since this product is a granulated and crushed product, powder dispersion in air can be prevented, and the product can be easily handled even in cases where it is in a small amount. Furthermore, since the product contains trehalose and the probiotics, it enables reduction of harmful microbial cells in the intestinal flora of poultry, and improvement of the productivity of the poultry.

### EXAMPLE 7

A feed for livestock during the sucking period was prepared by adding 10.0 g of trehalose (trade name "TREHA", commercially available from Hayashibara Co., Ltd.) and 10.0 g of probiotics (trade name "CALSPORIN", containing as an effective component the *Bacillus subtilis* C-3102 strain (FERM BP-1096) at 1.0×10¹⁰ cells/g-probiotics; commercially available from Asahi Biocycle Co., Ltd.) (corresponding to a number of live microbial cells of 1.0×10⁹ cells/g-feed) to 1 kg of a commercially available milk substitute. Since this feed contains trehalose and the probiotics, feeding of a cow or pig with the feed during the sucking period enables reduction of harmful microbial cells in the intestinal flora of the livestock, and improvement of the productivity of the livestock.

### EXAMPLE 8

A feed for livestock was prepared by adding 10.0 g of trehalose (trade name "TREHA", commercially available from Hayashibara Co., Ltd.) and 10.0 g of probiotics (trade name "CALSPORIN", containing as an effective component the *Bacillus subtilis* C-3102 strain (FERM BP-1096) at 1.0×10¹⁰ cells/g- probiotics; commercially available from Asahi Biocycle Co., Ltd.) (corresponding to a number of live microbial cells of 1.0×10⁹ cells/g-feed) to 1 kg of a concentrated feed. Since this feed contains trehalose and the probiotics, feeding of a cow or pig with the feed during the fattening period enables reduction of harmful microbial cells in the intestinal flora of the livestock, and improvement of the productivity of the livestock.

### INDUSTRIAL APPLICABILITY

The method of improving the productivity of livestock and/or poultry, and the method of improving the intestinal flora of livestock and/or poultry, of the present invention enable improvement of the intestinal flora of poultry, and improvement of the weight gain, the feed conversion ratio, and the uniformity of livestock and/or poultry. Since the present invention provides the method of improving the productivity of livestock and/or poultry, and the method of improving the intestinal flora of livestock and/or poultry, the invention contributes to the field of animal husbandry, and plays a significant role in the industry.

### DESCRIPTION OF SYMBOLS

The following are terms in Fig. 1.
Starter feed: an early-stage feed for feeding from 0 day after birth to 10 days old in the early rearing period (chick feed)
Grower feed: a feed for promoting the growth, for feeding from 10 days old to 24 days old in the early rearing period (growth-promoting feed)
Finisher feed: a feed for feeding from 24 days old in the late rearing period (late-stage feed)

## Claims

1. A method of improving productivity of livestock and/or poultry, the method comprising feeding the livestock and/or poultry with a feed containing trehalose and probiotics.

2. The method of improving productivity of livestock and/or poultry according to claim 1, wherein the improvement of productivity is one or more selected from body weight gain, reduction in a feed conversion ratio, and improvement of uniformity.

3. A method of improving intestinal flora of livestock and/or poultry, the method comprising feeding the livestock and/or poultry with a feed containing trehalose and probiotics.

4. The method according to any one of claims 1 to 3, wherein the livestock and/or poultry is/are a cow, pig, and/or chicken.

5. The method according to any one of claims 1 to 4, wherein the probiotics comprise a bacterium belonging to the genus *Bacillus.*

6. The method according to claim 5, wherein the bacterium belonging to the genus *Bacillus* is *Bacillus subtilis.*

7. The method according to any one of claims 1 to 6, wherein the feeding with the feed containing trehalose and probiotics is carried out during an early rearing period.

8. The method according to any one of claims 1 to 7, wherein the feed is a feed comprising 0.05% by mass to 2% by mass trehalose per feed solid, and 3.0×10⁵ to 1.0×10⁹ live microbial cells per 1 g (gram) of feed solid.

9. A feed for livestock and/or poultry, the feed comprising trehalose and probiotics.

10. The feed for livestock and/or poultry according to claim 9, wherein the probiotics comprise a bacterium belonging to the genus *Bacillus.*

11. The feed for livestock and/or poultry according to claim 10, wherein the bacterium belonging to the genus *Bacillus* is *Bacillus subtilis.*

12. The feed for livestock and/or poultry according to any one of claims 9 to 11, comprising 0.05% by mass to 2% by mass trehalose per feed solid, and 3.0×10⁵ to 1.0×10⁹ live microbial cells per 1 g of feed solid.

13. The feed for livestock and/or poultry according to any one of claims 9 to 12, which is a feed for an early rearing period.
